# EUROPÄISCHE PATENTSCHRIFT

(11) **EP 3 046 541 B1**
(45) Veröffentlichungstag und Bekanntmachung des Hinweises auf die Patenterteilung: **31.10.2018**
(21) Anmeldenummer: 14771832.4
(22) Anmeldetag: 16.09.2014
(51) Int. Cl.: A61K 9/127, A61K 33/06

(54) **MAGNESIUM-LIPOSOMEN**
MAGNESIUM-LIPOSOMES
LIPOSOMES CONTENANT DU MAGNÉSIUM

(30) Priorität: 17.09.2013 DE 102013015334
(43) Veröffentlichungstag der Anmeldung: 27.07.2016
(73) Patentinhaber: Fresenius Medical Care Deutschland GmbH, 61352 Bad Homburg (DE)
(72) Erfinder: BÜCHEL, Janine, 61350 Bad Homburg (DE); HOFMANN, Wolfgang, 60487 Frankfurt am Main (DE); GUNDLACH, Kristina, 55126 Mainz (DE); YILLAH, Jasmin, 60433 Frankfurt am Main (DE); STEPPAN, Sonja, 63263 Neu-Isenburg (DE); ARENS, Hans-Jürgen, 61440 Oberursel (DE)
(74) Vertreter: Stolmár & Partner Patentanwälte PartG mbB
(86) Internationale Anmeldenummer: PCT/EP2014/069729
(87) Internationale Veröffentlichungsnummer: WO 2015/040028

(56) Entgegenhaltungen:
- WO-A1-88/07852
- US-A1- 2010 003 315
- ANNE S ULRICH: "Biophysical aspects of using liposomes as delivery vehicles", BIOSCIENCE REPORTS, Bd. 22, Nr. 2, 1. April 2002 (2002-04-01), Seiten 129-150, XP55053119, ISSN: 0144-8463, DOI: 10.1023/A:1020178304031
- KLIBANOV A L ET AL: "Amphipathic polyethyleneglycols effectively prolong the circulation time of liposomes", FEBS LETTERS, ELSEVIER, AMSTERDAM, NL, Bd. 268, Nr. 1, 30. Juli 1990 (1990-07-30) , Seiten 235-237, XP025890437, ISSN: 0014-5793, DOI: 10.1016/0014-5793(90)81016-H [gefunden am 1990-07-30]
- SALES ET AL: "Magnesium and diabetes mellitus: Their relation", CLINICAL NUTRITION, CHURCHILL LIVINGSTONE, LONDON, GB, Bd. 25, Nr. 4, 1. August 2006 (2006-08-01) , Seiten 554-562, XP005560056, ISSN: 0261-5614
- WHELTON P K ET AL: "Magnesium and blood pressure: Review of the epidemiologic and clinical trial experience", AMERICAN JOURNAL OF CARDIOLOGY, CAHNERS PUBLISHING CO., NEWTON, MA, US, Bd. 63, Nr. 14, 18. April 1989 (1989-04-18), Seiten G26-G30, XP023207970, ISSN: 0002-9149, DOI: 10.1016/0002-9149(89)90215-4 [gefunden am 1989-04-18]
- MARK HOUSTON: "The Role of Magnesium in Hypertension and Cardiovascular Disease", THE JOURNAL OF CLINICAL HYPERTENSION, Bd. 13, Nr. 11, 26. September 2011 (2011-09-26), Seiten 843-847, XP55150708, ISSN: 1524-6175, DOI: 10.1111/j.1751-7176.2011.00538.x

## Beschreibung

### Technisches Gebiet

Die vorliegende Erfindung betrifft Magnesium-Liposomen mit verzögerter Magnesiumfreigabe und Verfahren zu ihrer Herstellung. Weiterhin betrifft die vorliegende Erfindung Magnesium-Liposomen zur Verwendung in der Behandlung von Krankheiten, die mit einem Magnesiummangel einhergehen.

Magnesium ist das vierthäufigste Kation im menschlichen Körper und Co-Enzym von über 300 Enzymen. Magnesium ist wichtig für die Bildung von ATP und Nukleinsäuren sowie für die Muskelfunktion.

Trotz seiner Bedeutung sind viele Patienten unerkannt hypomagnesemisch, haben also einen zu geringen Magnesiumspiegel.

Dies kann klinische Auswirkungen haben, denn Hypomagnesämie ist assoziiert mit Diabetes mellitus, Dyslipidämie, endothelialer Dysfunktion, oxidativem Stress, Asthma, Bluthockdruck und Inflammation. Besonders Inflammation in den Gefäßen und Gefäßverkalkung führen zu kardiovaskulären Problemen und können einen frühzeitigen Tod verursachen. Krankhafte kardiovaskuläre Veränderungen sind weit verbreitet und mit einer Vielzahl anderer Erkrankungen assoziiert. Bei Patienten mit chronischen Nierenerkrankungen (CKD [chronic kidney disease] Patienten) stellen sie beispielsweise die häufigste Todesursache dar.

Bei CKD-Patienten konnte gezeigt werden, dass höhere Magnesiumwerte mit geringerer Verkalkung und Mortalität assoziiert sind. Darüber hinaus inhibiert Magnesium ungewollte Verkalkung durch einen direkten zellulären Effekt.

Die erforderliche Tagesdosis von circa 300 mg wird in der Regel durch eine ausgewogene Ernährung erreicht. Ein erhöhter Bedarf kann über Nahrungsergänzungsmittel oder Medikamente gedeckt werden. Dies kann zum Beispiel durch Tabletten, insbesondere Brausetabletten, geschehen.

Bei CKD-Patienten ergibt sich bei oraler Gabe von Magnesium jedoch das Problem der Compliance. Eine wirksame Kontrolle der Einnahme ist nicht möglich, da die Tabletten zu Hause ohne ärztliche Aufsicht eingenommen werden. Nierenkranke Patienten müssen pro Tag bereits mindestens 12 Tabletten, wie beispielsweise Eisenpräparate, Phosphat- und Kaliumbinder, einnehmen. Eine Tagesmenge von bis zu 30 Tabletten ist nicht ungewöhnlich. Dabei dürfen sie nur maximal einen Liter Wasser trinken. Dies hat zur Folge, dass viele Tabletten gar nicht eingenommen werden.

Darüber hinaus führt die orale Gabe von Magnesium zu gastrointestinalen Unregelmäßigkeiten. Die schlecht resorbierbaren Magnesium-Ionen führen im Darm zu einem osmotischen Gradienten, der einen Flüssigkeitseinstrom in den Verdauungstrakt zur Folge hat. Dies erhöht die Fließfähigkeit des Darminhaltes und hat im Ergebnis eine abführende Wirkung.

Eine weitere Möglichkeit besteht darin, Magnesium in Form einer sterilen Magnesiumsulfatlösung intravenös zu verabreichen. In der Notfallmedizin wird dies beispielsweise zur Behandlung einer Eklampsie eingesetzt.

Gefürchtete Nebenwirkung einer intravenösen Verabreichung von Magnesium ist der Atemstillstand. Er kündigt sich zumeist durch Ausfall der Muskeleigenreflexe an (Areflexie). Deshalb sollte Magnesium nur sehr langsam intravenös injiziert werden. Weitere Nebenwirkungen sind Kopfschmerzen, Herzklopfen, Schwindel, Übelkeit und Erbrechen.

Eine intravenöse Verabreichung einer Magnesiumsulfatlösung führt zu einer kurzzeitigen, starken Erhöhung des Serummagnesiumspiegels, der dann jedoch rasch wieder abfällt. Eine Bolusgabe von Magnesiumsulfat ist also nicht geeignet den Magnesiumspiegel im menschlichen Körper über einen längeren Zeitraum, beispielsweise über Stunden oder Tage, zu erhöhen.

Aus dem Stand der Technik ist bekannt, Arzneistoffe in Liposome einzuschließen. Durch die liposomale Formulierung von Arzneistoffen ist es möglich, lipophile Wirkstoffe bioverfügbar zu machen. Zudem können empfindliche Arzneistoffe durch Einschluss in Liposomen vor einer möglichen Metabolisierung nach der Applikation geschützt und so die Plasmahalbwertszeit des Wirkstoffs erhöht werden.

Liposomen als "Drug Delivery System" ermöglichen einen zielgerichteten und selektiven Transport von Arzneistoffen an jene Stellen im Organismus, an denen sie benötigt werden. Dadurch werden Nebenwirkungen des liposomal formulierten Arzneistoffes verringert und, da niedrigere Dosen verabreicht werden können, die Effizienz und die therapeutische Breite erhöht. Hydrophile Arzneistoffe werden umschlossen und befinden sich im hydrophilen Inneren des Liposoms. Lipophile Arzneistoffe werden in die Membran eingebettet und amphiphile Arzneistoffe finden sich an der Grenzfläche zwischen dem Membraninneren und der wässrigen Phase ein.

Magnesium-Liposomen sind aus dem Stand der Technik bekannt.

US 5,501,859 offenbart eine absorbierbare Magnesiumzusammensetzung zur oralen Verabreichung umfassend ein Magnesiumsalz und Liposomen aus 1-Palmitoyl-2-oleoyl-phosphatidylcholin.

WO 88/07852 offenbart positiv geladene, unilamellare Liposomen enthaltend Magnesium zur intravenösen, intramuskulären oder subkutanen Verabreichung. Positiv geladene Liposomen sind jedoch toxisch und werden rasch durch das mononukleare Phagozytensystem erkannt und aus dem Blutkreislauf entfernt. Positiv geladene Liposomen sind somit ebenfalls nicht geeignet, den Magnesiumspiegel im menschlichen Körper über einen längeren Zeitraum, also beispielsweise über Stunden oder Tage, zu erhöhen.

US 2010/0003315 offenbart die Verwendung von Liposomen, die mit Magnesium angereichertes Meerwasser enthalten, zur Anwendung auf der Haut, um beispielsweise Hautkrankheiten, wie Akne, zu behandeln.

### Beschreibung der Erfindung

Die vorliegende Erfindung betrifft Magnesium-Liposomen mit verzögerter Magnesiumfreigabe zur intravenösen Verabreichung. Die Ansprüche offenbaren Magnesium-Liposomen dadurch gekennzeichnet, dass die Liposomen zumindest teilweise mit einer Polyalkylenglykol- oder Polyglycerolverbindung beschichtet sind und dass die Ladung der Magnesium-Liposomen neutral ist.

Unter Magnesium-Liposomen werden Liposomen verstanden, die Magnesium in Form eines Salzes enthalten.

Das in die Liposomen eingebaute Magnesiumsalz umfasst jedes pharmazeutisch verträgliche Magnesiumsalz erhältlich aus der Reaktion von Magnesium mit einer Säure ausgewählt aus Salzsäure, Bromwasserstoffsäure, Phosphorsäure, Salpetersäure, Schwefelsäure, Essigsäure, 2,2-Dichloressigsäure, Adipinsäure, Ascorbinsäure (D-oder L-Form davon, insbesondere die L-Form davon), Asparaginsäure (D-oder L-Form davon, insbesondere die L-Form davon), Benzolsulfonsäure, Benzoesäure, 4-Acetamido-benzoesäure, Camphersäure ((+) - oder (-) - Form davon, insbesondere das (+) - Form davon), Campher-10-sulfonsäure ((+) - oder (-) - Form davon, insbesondere das (+) - Form davon), Caprinsäure (Decansäure), Capronsäure (Hexansäure), Caprylsäure (Octansäure), Kohlensäure, Zimtsäure, Citronensäure, Cyclaminsäure Dodecylschwefelsäureesters, Ethan-1 ,2-disulfonsäure, Ethansulfonsäure, 2 - Hydroxy-Ethansulfonsäure, Ameisensäure, Fumarsäure, Galactarsäure, Gentisinsäure, Glucoheptonsäure (D-oder L-Form davon, insbesondere die D-Form davon), Gluconsäure (D-oder L-Form davon, insbesondere die D-Form davon); Glucuronsäure (D-oder L-Form davon, insbesondere die D-Form davon), Glutaminsäure, Glutarsäure, 2-Oxo-Glutarsäure, Glycerophosphorsäure, Glycolsäure, Hippursäure, Isobuttersäure, Milchsäure (D-oder L-Form davon), Lactobionsäure, Laurinsäure, Maleinsäure, Apfelsäure (D- oder L-Form davon), Malonsäure, Mandelsäure (D- oder L-Form davon), Methansulfonsäure, Naphthalin-1, 5 -disulfonsäure, Naphthalin-2-sulfonsäure, 1-Hydroxy-2-naphthoesäure, Nikotinsäure, Ölsäure, Orotsäure, Oxalsäure, Palmitinsäure, Pamoasäure (Embonsäure), Propionsäure, Pyroglutaminsäure (D- oder L-Form davon, insbesondere die L-Form davon), Salicylsäure, 4-Aminosalicylsäure, Sebacinsäure, Stearinsäure, Bernsteinsäure, Weinsäure (D- oder L-Form davon), Thiocyansäure, Toluolsulfonsäure (insbesondere das *p*-Isomer) und Undecylensäure oder deren Solvate, Hydrate oder deren Mischungen.

Vorzugsweise ist das Magnesiumsalz wasserlöslich. Besonders bevorzugt sind Magnesiumsulfat und Magnesiumchlorid.

Die Magnesium-Liposomen sind multi- oder unilamellar aufgebaut und umfassen Cholesterin, Phosphatidylcholine, Phosphatidylethanolamine, Phosphatidylserine, sowie weitere Bestandteile, wie beispielsweise Fettsäuren sind Olein-, Palmitin- und Linolensäure.

Mischungen von Phosphatidylcholinen können aus beispielsweise aus Hühnerei (Ei-Phosphatidylcholin, E-PC) oder Soja gewonnen werden. Weitere Beispiele für Phosphatidylcholine sind 1,2-Diphytanoyl-*sn-*glycero-3-phosphocholin (DPhyPC), 1,2-Dipalmitoyl-*sn*-glycero-3-phosphocholin (DPPC), 1,2-Dilauroyl-*sn*-glycero-3-phosphocholin (DLPC), 1,2-Dioleoyl-*sn*-glycero-3-phosphocholin (DOPC), 1,2-Dimyristoyl-*sn-*glycero-3-phosphocholin (DMPC), 1,2-(cis-cis-9,12-Octadecadienoyl)-*sn-*glycero-3-phosphatidylcholin (DUPC), 1-Palmitoyl-2-linoleoyl-*sn*-glycero-3-phosphocholin (PLPC), 1-Palmitoyl-2-oleoyl-*sn*-glycero-3-phosphocholin (POPC), 1-Stearoyl-2-oleoyl-*sn*-glycero-3-phosphocholin (SOPC).

Beispiele für Phosphatidylethanolamine sind 1,2-Dimyristoyl-*sn*-glycero-3-phosphoethanolamin (DMPE), 1,2-Dilauroyl-*sn-*glycero-3-phosphoethanolamin (DLPE), 1,2-Dioleoyl-*sn*-glycero-3-phosphoethanolamin (DOPE), 1,2-Dipalmitoyl-*sn*-glycero-3-phosphoethanolamin (DPPE), 1-Palmitoyl-2-oleoyl-*sn*-glycero-3-phosphoethanolamin (POPE).

Ein Beispiel für ein Phosphatidylserin ist 1,2-di-(9Z-octadecenoyl)-*sn-*glycero-3-phospho-L-serine (DOPS)

Verfahren zur Herstellung von Liposomen sind dem Fachmann geläufig. Unilamellare Liposomen lassen sich beispielsweise durch langsames Eintropfen von ethanolischen oder etherhaltigen Lipidlösungen in die wässrige Phase herstellen. Weitere Verfahren sind die Dialyse von Detergens-Lipidgemischen oder die Ultrabeschallung und Vibration von wässrigen Lösungen auf zuvor getrockneten Lipidfilmen. Das bevorzugte Verfahren zur Herstellung von Liposomen, insbesondere Multilamellaren Vesikeln (MLV), umfasst den Schritt der Extrusion der Lipidmischung durch Membranen bestimmter Porengröße (z.B. 100 nm), beispielsweise einer Polycarbonatmembran, oder die Hochdruckhomogenisation.

Die Ladung der erfindungsgemäßen Magnesium-Liposomen ist neutral. Es wurde gefunden, dass neutrale Magnesium-Liposomen im Vergleich zu positiv oder negativ geladenen Liposomen eine geringere Komplementaktivierung hervorrufen und damit deren Halbwertszeit im Blutkreislauf verlängert wird.

Die erfindungsgemäßen Magnesium-Liposomen haben einen mittleren Durchmesser von kleiner 400 nm, vorzugsweise 70 bis 250 nm. Ein mittlerer Durchmesser von 100 bis 200 nm ist besonders bevorzugt, da Magnesium-Liposomen auf der einen Seite klein genug sind, um in der Leber oder von Makrophagen nur verzögert aufgenommen zu werden, auf der anderen Seite noch genug Raum für das Magnesiumsalz bieten.

Die erfindungsgemäßen Magnesium-Liposomen umfassen sowohl nicht modifizierte Liposomen als auch Liposomen, welche mittels einer Polyalkylenglykolkette oder eines Polyglycerols modifiziert wurden. Als das Polyalkylenglykol können zum Beispiel Polyethylenglykol (PEG), Polypropylenglykol oder dergleichen verwendet werden. In einer bevorzugten Ausführungsform ist das Polyalkylenglykol Polyethylenglykol. Die Oberflächenmodifizierung von Liposomen mit beispielsweise Polyethylenglykol kann auf verschiedenen Wegen erfolgen. Bevorzugt wird Polyethylenglykol mittels kovalenter chemischer Bindung auf der Oberfläche von Liposomen verankert. Hierfür bieten sich zwei Syntheserouten an. Zum einen kann Polyethylenglykol auf fertigen Liposomen verankert werden, zum anderen besteht die Möglichkeit, in einem ersten Schritt Polyethylenglykol-Lipid-Konjugate zu synthetisieren und diese in einem zweiten Schritt bei der Herstellung der Liposomen zu verwenden. Ein Beispiel für ein solches Polyethylenglykol-Konjugat ist Poly-(ethylenglykol)-phosphatidylethanolamin (PEG-PE).

Wenn Polyethylenglykol verwendet wird, können solche mit einem Molekulargewicht bis etwa 7000 Dalton, vorzugsweise zwischen 1000 bis 5000 Dalton, verwendet werden.

Die Menge der Polyalkylenglykolverbindung, die zur Modifikation der Magnesium-Liposomen verwendet wird, ist nicht besonders begrenzt. Eine bevorzugt eingesetzte Menge des Polyalkylenglykols ist, bezogen auf die gesamten Lipide, ungefähr 0,2 bis 5 Mol-%, bevorzugt ungefähr 0,28 bis 3 Mol-%, besonders bevorzugt 0,3 bis 2,5 Mol-%

Die vorliegende Erfindung betrifft weiterhin pharmazeutische Formulierungen, die zur intravenösen Verabreichung der erfindungsgemäßen Magnesium-Liposomen geeignet sind. In einer bevorzugten Ausführungsform der vorliegenden Erfindung sind die erfindungsgemäßen Magnesium-Liposomen lyophilisiert. Pharmazeutische Formulierungen enthaltend die erfindungsgemäßen Magnesium-Liposomen können weitere Hilfsstoffe umfassen, wie beispielsweise Disaccharide, insbesondere Saccharose, Lactose oder Trehalose.

Die lyophilisierten Magnesium-Liposomen können in Ampullen, Vials oder Durchstechflaschen abgefüllt sein. Vor der Verabreichung wird ein geeignetes Suspendierungsmedium, wie beispielsweise isotonische Kochsalzlösung, hinzugefügt und eine Liposomen-Suspension hergestellt.

Die vorliegende Erfindung betrifft weiterhin ein Dosierungsschema zur Verabreichung von Magnesium-Liposomen, welches den Abbau der Magnesium-Liposomen durch das mononukleare Phagozytensystem verzögert.

Der Abbau kann beispielweise dadurch verzögert werden, dass Liposomen mit einer negativen Ladung oder solche ohne beschichtete Oberfläche oder "leere" Liposomen, also Liposomen ohne Magnesiumsalz, vorab verabreicht werden und das mononukleare Phagozytensystem auf diese Weise sättigen. Anschließend werden dann die magnesiumhaltigen Liposomen verabreicht.

In einer weiteren Ausführungsform werden "leere" und magnesiumhaltige Liposomen gleichzeitig verabreicht. Das Stoffmengenverhältnis der "leeren" und magnesiumhaltigen Liposomen beträgt dabei 10:1 bis 1:10, bevorzugt 5:1 bis 1:2, besonders bevorzugt 2:1 bis 1:1.

Eine weitere, bevorzugte Ausführungsform betrifft eine pharmazeutische Formulierung enthaltend magnesiumfreie und magnesiumhaltige Liposomen in einem Stoffmengenverhältnis von 10:1 bis 1:10, bevorzugt 5:1 bis 1:2, besonders bevorzugt 2:1 bis 1:1.

Die erfindungsgemäßen Magnesium-Liposomen eignen sich zur Verwendung in der Prävention und Behandlung von Krankheiten, die mit Hypomagnesämie assoziiert sind.

Die erfindungsgemäßen Magnesium-Liposomen eignen sich zur Verwendung in der Behandlung von Patienten mit erhöhtem Risiko an kardiovaskulären Erkrankungen, insbesondere CKD-Patienten, unabhängig von ihrem Serummagnesiumspiegel.

Die erfindungsgemäßen Magnesium-Liposomen eignen sich insbesondere zur Verwendung in der Prävention und Behandlung von entzündlichen Gefäßerkrankungen.

Die erfindungsgemäßen Magnesium-Liposomen eignen sich insbesondere zur Verwendung in der Prävention und Behandlung von vaskulären Kalzifizierungen.

Die erfindungsgemäßen Magnesium-Liposomen eignen sich zur Verwendung in der Behandlung von Patienten mit metabolischem Syndrom, Typ 2 Diabetes mellitus (non-insulin dependent diabetes mellitus (NIDDM)).

Die erfindungsgemäßen Magnesium-Liposomen eignen sich zur Verwendung in der Prävention und Behandlung von Präeklampsie oder Eklampsie.

Die erfindungsgemäßen Magnesium-Liposomen eignen sich zur Verwendung in der Prävention und Behandlung von erhöhtem Blutdruck.

Die erfindungsgemäßen Magnesium-Liposomen eignen sich zur Verwendung in der Prävention und Behandlung des Fatigue-Syndroms.

### Beispiele

Die vorliegende Erfindung wird unter Bezugnahme auf die Beispiele erläutert.

### Beispiel 1: Herstellung eines Liposoms und Einkapseln eines Magnesiumsalzes

In Chloroform (1 ml) werden unter Rühren Ei-Phosphatidylcholin (E-PC, 10 mg) und Cholesterin (2 mg) gelöst. Anschließend wird das Chloroform im Rotationsverdampfer entfernt. Anschließend wird eine MgCl₂-Lösung (1 ml, Konzentration 1 mol/l) zugegeben und 40 min. bei Raumtemperatur im Rundkolben drehend hydratisiert. Die erhaltene Mischung wird 21 Mal durch eine Polycarbonatmembran (Porengröße 200 nm) extrudiert.

### Beispiel 2: Synthese von PEG-geschützen Magnesium-Liposomen aus Poly-(ethylenglykol)-phosphatidylethanolamin (PEG-PE)

Eine Lipidmischung (3,2 g) enthaltend Phosphatidylcholin (PC) Cholesterin, und Poly-(ethylenglykol)-phosphatidylethanolamin (PEG-PE) im molaren Verhältnis 18:10:0,5 wird in 32 ml 0,3 M Citratpuffer (pH 4,0) gegeben und hydratisiert. Dann wird durch dreimaliges Einfrieren und Auftauen der Mischung unter Verwendung von flüssigem Stickstoff und einem Wärmebad (60°C) multilamellare Liposome hergestellt. Anschließend wird eine wässrige Magnesiumchloridlösung in einer Menge von 0,5 ml von 20 mg/ml pro 100 mg Lipide für die Einkapselung hinzugegeben. Die Suspensionen werden wird mehrmals durch eine isopore Polykarbonatmembranen mit einer Porengröße von 200 nm extrudiert. Die sich ergebende Liposomensuspension wird durch tropfenweise Zugabe von 1 M NaOH neutralisiert.

## Patentansprüche

1. Magnesium-Liposomen **dadurch gekennzeichnet, dass** die Liposomen zumindest teilweise mit einer Polyalkylenglykol- oder Polyglycerolverbindung beschichtet sind und dass die Ladung der Magnesium-Liposomen neutral ist.

2. Magnesium-Liposomen nach Anspruch 1, wobei die Polyalkylenglykolverbindung Polyethylenglykol ist.

3. Magnesium-Liposomen nach einem der vorhergehenden Ansprüche, wobei das mittlere Molekulargewicht der Polyethylenglykolreste 500 bis 7000 Dalton, vorzugsweise 1000 bis 5000 Dalton beträgt.

4. Magnesium-Liposomen nach einem der vorhergehenden Ansprüche, wobei die mittlere Liposomengröße 70 bis 250 nm, bevorzugt 100 bis 250 nm, besonders bevorzugt 100 bis 200 nm beträgt.

5. Magnesium-Liposomen nach einem der vorhergehenden Ansprüche, wobei die Liposomen ein Magnesiumsalz umfassen, das erhältlich ist aus der Reaktion von Magnesium mit einer Säure ausgewählt aus Salzsäure, Bromwasserstoffsäure, Phosphorsäure, Salpetersäure, Schwefelsäure, Essigsäure, 2,2-Dichloressigsäure, Adipinsäure, Ascorbinsäure (D-oder L-Form davon , insbesondere die L-Form davon), Asparaginsäure (D-oder L-Form davon, insbesondere die L-Form davon), Benzolsulfonsäure, Benzoesäure, 4-Acetamido-benzoesäure, Camphersäure ((+)- oder (-)-Form davon, insbesondere das (+)-Form davon), Campher-10-sulfonsäure ((+)- oder (-)-Form davon, insbesondere das (+)-Form davon), Caprinsäure (Decansäure), Capronsäure (Hexansäure), Caprylsäure (Octansäure), Kohlensäure, Zimtsäure, Citronensäure, Cyclaminsäure Dodecylschwefelsäureesters, Ethan-1,2-disulfonsäure, Ethansulfonsäure, 2-Hydroxy-Ethansulfonsäure, Ameisensäure, Fumarsäure, Galactarsäure, Gentisinsäure, Glucoheptonsäure (D-oder L-Form davon, insbesondere die D-Form davon), Gluconsäure (D-oder L-Form davon, insbesondere die D-Form davon); Glucuronsäure (D-oder L-Form davon, insbesondere die D-Form davon), Glutaminsäure, Glutarsäure, 2-Oxo-Glutarsäure, Glycerophosphorsäure, Glycolsäure, Hippursäure, Isobuttersäure, Milchsäure (D-oder L-Form davon), Lactobionsäure, Laurinsäure, Maleinsäure, Apfelsäure (D-oder L-Form davon), Malonsäure, Mandelsäure (D-oder L-Form davon), Methansulfonsäure,
Naphthalin-1,5-disulfonsäure, Naphthalin-2-sulfonsäure, 1-Hydroxy-2-naphthoesäure, Nikotinsäure, Ölsäure, Orotsäure, Oxalsäure, Palmitinsäure, Pamoasäure (Embonsäure), Propionsäure, Pyroglutaminsäure (D- oder L-Form davon, insbesondere die L-Form davon), Salicylsäure, 4-Aminosalicylsäure, Sebacinsäure, Stearinsäure, Bernsteinsäure, Weinsäure (D-oder L-Form davon), Thiocyansäure, Toluolsulfonsäure (insbesondere das p-Isomer) und Undecylensäure oder deren Solvate, Hydrate oder deren Mischungen.

6. Pharmazeutische Zusammensetzung enthaltend Magnesium-Liposomen nach einem der vorhergehenden Ansprüche.

7. Pharmazeutische Zusammensetzung nach Anspruch 6, wobei die Magnesium-Liposomen lyophilisiert sind.

8. Pharmazeutische Formulierung nach Anspruch 6 oder 7, wobei die Zusammensetzung zusätzlich magnesiumfreie Liposomen enthält.

9. Verfahren zur Herstellung von Magnesium-Liposomen nach einem der vorhergehenden Ansprüche umfassend die Schritte:
a) Herstellung einer Lipidmischung,
b) Verkapseln eines Magnesiumsalzes,
c) Extrudieren oder Hochdruckhomogenisieren der Lipidmischung.

10. Magnesium-Liposomen nach einem der vorhergehenden Ansprüche zur Verwendung in der Behandlung einer mit Hypomagnesämie assoziierten Krankheit.

11. Magnesium-Liposomen nach einem der vorhergehenden Ansprüche zur Verwendung in der Behandlung von kardiovaskulären Erkrankungen, insbesondere bei CKD-Patienten.

12. Magnesium-Liposomen nach einem der vorhergehenden Ansprüche zur Verwendung in der Behandlung des metabolischen Syndroms oder Typ 2 Diabetes mellitus (non-insulin dependent diabetes mellitus (NIDDM)).

13. Magnesium-Liposomen nach einem der vorhergehenden Ansprüche zur Verwendung in der Prävention und Behandlung von vaskulären Kalzifizierungen.

14. Magnesium-Liposomen nach einem der vorhergehenden Ansprüche zur Verwendung in der Prävention und Behandlung von Präeklampsie oder Eklampsie.

15. Magnesium-Liposomen nach einem der vorhergehenden Ansprüche zur Verwendung in der Prävention und Behandlung von erhöhtem Blutdruck.

16. Magnesium-Liposomen nach einem der vorhergehenden Ansprüche zur Verwendung in der Behandlung des Fatigue-Syndroms.

## Claims

1. Magnesium liposomes **characterized in that** the liposomes are at least partially coated with a polyalkylene glycol or polyglycerol compound and **in that** the charge of the magnesium liposomes is neutral.

2. Magnesium liposomes according to claim 1, wherein the polyalkylene glycol compound is polyethylene glycol.

3. Magnesium liposomes according to any one of the preceding claims, wherein the average molecular weight of the polyethylene glycol residues is 500 to 7000 daltons, preferably 1000 to 5000 daltons.

4. Magnesium liposomes according to one of the preceding claims, wherein the mean liposome size is 70 to 250 nm, preferably 100 to 250 nm, particularly preferably 100 to 200 nm.

5. Magnesium liposomes according to any one of the preceding claims, wherein the liposomes comprise a magnesium salt obtainable from the reaction of magnesium with an acid selected from hydrochloric acid, hydrobromic acid, phosphoric acid, nitric acid, sulfuric acid, acetic acid, 2,2-dichloroacetic acid, adipic acid, ascorbic acid (D- or L-form thereof, in particular the L-form thereof), aspartic acid (D- or L-form thereof, in particular the L-form thereof), benzenesulfonic acid, benzoic acid, 4-acetamido-benzoic acid, camphoric acid ((+)- or (-)-form thereof, in particular the (+)-form thereof), camphor-10-sulfonic acid ((+)- or (-)- form thereof, in particular the (+)-form thereof), capric acid (decanoic acid), caproic acid (hexanoic acid), caprylic acid (octanoic acid), carbonic acid, cinnamic acid, citric acid, cyclamic acid dodecylsulfuric acid esters, ethane-1,2-disulfonic acid, ethanesulfonic acid, 2-hydroxy-ethanesulfonic acid, formic acid, fumaric acid, galactaric acid, gentisic acid, glucoheptonic acid (D- or L-form thereof, in particular the D-form thereof); gluconic acid (D- or L-form thereof, in particular the D-form thereof); glucuronic acid (D- or L-form thereof, in particular the D-form thereof), glutamic acid, glutaric acid, 2-oxo-glutaric acid, glycerophosphoric acid, glycolic acid, hippuric acid, isobutyric acid, lactic acid (D- or L-form thereof), lactobionic acid, lauric acid, maleic acid, malic acid (D- or L-form thereof), malonic acid, mandelic acid (D- or L-form thereof), methanesulfonic acid, naphthalene-1,5-disulfonic acid, naphthalene-2-sulfonic acid, 1-hydroxy-2-naphthoic acid, nicotinic acid, oleic acid,
orotic acid, oxalic acid, palmitic acid, pamoic acid (embonic acid), propionic acid, pyroglutamic acid (D- or L-form thereof, in particular the L-form thereof), salicylic acid, 4-aminosalicylic acid, sebacic acid, stearic acid, succinic acid, tartaric acid (D- or L-form thereof), thiocyanic acid, toluenesulfonic acid (in particular the p-isomer) and undecylenic acid or their solvates, hydrates or mixtures thereof.

6. Pharmaceutical composition containing magnesium liposomes according to one of the preceding claims.

7. The pharmaceutical composition of claim 6, wherein the magnesium liposomes are lyophilized.

8. A pharmaceutical formulation according to claim 6 or 7, wherein the composition additionally contains magnesium-free liposomes.

9. Method for the preparation of magnesium liposomes according to any one of the preceding claims comprising the steps:
a) preparation of a lipid mixture,
b) encapsulating a magnesium salt,
c) extruding or high pressure homogenizing the lipid mixture.

10. Magnesium liposomes according to any one of the preceding claims for use in the treatment of a disease associated with hypomagnesaemia.

11. Magnesium liposomes according to one of the preceding claims for use in the treatment of cardiovascular diseases, in particular in CKD patients.

12. Magnesium liposomes according to any one of the preceding claims for use in the treatment of metabolic syndrome or type 2 diabetes mellitus (non-insulin dependent diabetes mellitus (NIDDM)).

13. Magnesium liposomes according to any one of the preceding claims for use in the prevention and treatment of vascular calcifications.

14. Magnesium liposomes according to any one of the preceding claims for use in the prevention and treatment of pre-eclampsia or eclampsia.

15. Magnesium liposomes according to any one of the preceding claims for use in the prevention and treatment of elevated blood pressure.

16. Magnesium liposomes according to any one of the preceding claims for use in the treatment of fatigue syndrome.

## Revendications

1. Liposomes de magnésium **caractérisés en ce que** les liposomes sont au moins partiellement revêtus d'un composé de polyalkylène glycol ou de polyglycérol et que la charge des liposomes de magnésium est neutre.

2. Liposomes de magnésium selon la revendication 1, dans lesquels le composé polyalkylène glycol est le polyéthylène glycol.

3. Liposomes de magnésium selon l'une quelconque des revendications précédentes, dans lesquels le poids moléculaire moyen des résidus de polyéthylène glycol est de 500 à 7000 daltons, de préférence de 1000 à 5000 daltons.

4. Liposomes de magnésium selon l'une des revendications précédentes, dans lesquels la taille moyenne des liposomes est de 70 à 250 nm, de préférence de 100 à 250 nm, de manière particulièrement préférée de 100 à 200 nm.

5. Liposomes de magnésium selon l'une quelconque des revendications précédentes, dans lequel les liposome comprennent un sel de magnésium, qui peut être obtenu à partir de la réaction du magnésium avec un acide choisi parmi l'acide chlorhydrique, l'acide bromhydrique, l'acide phosphorique, l'acide nitrique, l'acide sulfurique, l'acide acétique, l'acide 2,2-dichloroacétique, l'acide adipique, l'acide ascorbique (D- ou L-forme de celui-ci, en particulier la L-forme de celui-ci), l'acide aspartique (D- ou L-forme de celui-ci, en particulier la L-forme de celui-ci), l'acide benzènesulfonique, l'acide benzoïque, l'acide 4-acétamido-benzoïque, l'acide camphorique ((+)- ou (-)-forme de celui-ci, en particulier la (+)-forme de celui-ci), l'acide camphre-10-sulfonique ((+)- ou (-)-forme de celui-ci, en particulier la (+)-forme de celui-ci), l'acide caprique (acide décanoïque), l'acide caproïque (acide hexanoïque), l'acide caprylique (acide octanoïque), l'acide carbonique, l'acide cinnamique, l'acide citrique, l'acide cyclamique l'acide dodécylsulfate ester, l'acide éthane-1,2-disulfonique, l'acide éthanesulfonique, l'acide 2-hydroxy-éthanesulfonique, l'acide formique, l'acide fumarique, l'acide galactarique, l'acide gentisique, l'acide glucoheptonique (D- ou L-forme de celui-ci, en particulier la D-forme de celui-ci), l'acide gluconique (D-ou L-forme de celui-ci, en particulier la D-forme de celui-ci); l'acide glucuronique (D-ou L-forme de celui-ci, en particulier la D-forme de celui-ci), l'acide glutamique, l'acide glutarique, l'acide 2-oxo-glutarique, l'acide glycérophosphorique, l'acide glycolique, l'acide hippurique, l'acide isobutyrique, l'acide lactique (D- ou L-forme de celui-ci), l'acide lactobionique, l'acide laurique, l'acide maléique, l'acide malique (D-ou L-forme de celui-ci), l'acide malonique, l'acide mandélique (D-ou L-forme de celui-ci), l'acide méthanesulfonique, l'acide naphtalène-1,5-disulfonique, l'acide naphtalène-2-sulfonique, l'acide 1-hydroxy-2-naphtoïque, l'acide nicotinique, l'acide oléique, l'acide orotique, l'acide oxalique, l'acide palmitique, l'acide pamoïque (embonique), l'acide propionique, l'acide pyroglutamique (D- ou L-forme de celui-ci, en particulier la L-forme de celui-ci), l'acide salicylique, l'acide 4-aminosalicylique, l'acide sébacique, l'acide stéarique, l'acide succinique, l'acide tartrique (D-ou L-forme de celui-ci), l'acide thiocyanique, l'acide toluènesulfonique (en particulier l'isomère p) et l'acide undécylénique ou leurs solvates, hydrates ou leurs mélanges.

6. Composition pharmaceutique contenant des liposomes de magnésium selon l'une des revendications précédentes.

7. Composition pharmaceutique selon la revendication 6, dans laquelle les liposomes de magnésium sont lyophilisés.

8. Formulation pharmaceutique selon la revendication 6 ou 7, dans laquelle la composition contient en outre des liposomes sans magnésium.

9. Procédé de préparation de liposomes de magnésium selon l'une quelconque des revendications précédentes comprenant les étapes:
a) préparer un mélange de lipides,
b) encapsuler un sel de magnésium,
c) extrusion ou homogénéisation sous haute pression du mélange lipidique.

10. Liposomes de magnésium selon l'une quelconque des revendications précédentes destinés à être utilisés dans le traitement d'une maladie associée à l'hypomagnésémie.

11. Liposomes de magnésium selon l'une des revendications précédentes destinés à être utilisés dans le traitement de maladies cardiovasculaires, en particulier chez des patients atteints d'IRC.

12. Liposomes de magnésium selon l'une quelconque des revendications précédentes destinés à être utilisés dans le traitement du syndrome métabolique ou du diabète sucré de type 2 (diabète sucré non insulinodépendant (NIDDM)).

13. Liposomes de magnésium selon l'une quelconque des revendications précédentes destinés à être utilisés dans la prévention et le traitement des calcifications vasculaires.

14. Liposomes de magnésium selon l'une quelconque des revendications précédentes destinés à être utilisés dans la prévention et le traitement de la pré-éclampsie ou de l'éclampsie.

15. Liposomes de magnésium selon l'une quelconque des revendications précédentes destinés à être utilisés dans la prévention et le traitement d'une pression artérielle élevée.

16. Liposomes de magnésium selon l'une quelconque des revendications précédentes destinés à être utilisés dans le traitement du syndrome de fatigue.
